# EUROPEAN PATENT SPECIFICATION

(11) **EP 1 301 794 B1**
(45) Date of publication and mention of the grant of the patent: **01.04.2009**
(21) Application number: 01927036.2
(22) Date of filing: 16.04.2001
(51) Int. Cl.: G01N 33/68, G01N 33/574

(54) **METHOD FOR QUANTIFICATION OF AKT PROTEIN EXPRESSION**
VERFAHREN ZUR QUANTIFIZIERUNG DER AKT-PROTEIN-EXPRESSION
M THODE DE QUANTIFICATION DE L'EXPRESSION DE PROT INE AKT

(30) Priority: 14.04.2000 US 197780 P
(43) Date of publication of application: 16.04.2003
(73) Proprietor: VENTANA MEDICAL SYSTEMS, INC., Tucson, AZ 85705 (US); THE BOARD OF TRUSTEES OF THE UNIVERSITY OF ILLINOIS, Urbana, IL 61801 (US)
(72) Inventor: BACUS, Sarah, S., Elmhurst, IL 60126 (US); GUDKOV, Andrei, Glencoe, IL 60022 (US)
(74) Representative: Müller-Boré & Partner Patentanwälte
(86) International application number: PCT/US2001/012288
(87) International publication number: WO 2001/079855

(56) References cited:
- WO-A-01/79557
- US-A1- 2001 044 124
- US-A1- 2002 037 541
- BACUS SARAH S ET AL: "AKT2 is frequently upregulated in HER-2/neu-positive breast cancers and may contribute to tumor aggressiveness by enhancing cell survival." ONCOGENE. ENGLAND 16 MAY 2002, vol. 21, no. 22, 16 May 2002 (2002-05-16), pages 3532-3540, XP008005733 ISSN: 0950-9232
- BACUS SARAH S ET AL: "Akt2 upregulation in HER-2/neu overexpressing breast cancers: Implications to their clinical and biological behavior." PROCEEDINGS OF THE AMERICAN ASSOCIATION FOR CANCER RESEARCH ANNUAL, vol. 42, March 2001 (2001-03), page 243 XP008005731 92nd Annual Meeting of the American Association for Cancer Research;New Orleans, LA, USA; March 24-28, 2001, March, 2001 ISSN: 0197-016X

## Description

The invention relates to methods for the quantification of protein expression in cells or tissue samples related to diagnosis and treatment of disease states, particularly cancer disease states. Specifically, the invention relates to methods for the quantification of AKT1 and AKT2 protein expression as well as their activation state in which an imaging system is used to quantify AKT1 and AKT2 protein expression.

A cancer diagnosis is conventionally confirmed through histological examination of cell or tissue samples removed from a patient. Clinical pathologists need to be able to accurately determine whether such samples are benign or malignant and to classify the aggressiveness of tumor samples deemed to be malignant, because these determinations often form the basis for selecting a suitable course of patient treatment.

Histological examination traditionally entails tissue-staining procedures that permit the morphological features of a sample to be readily observed under a light microscope. A pathologist, after examining the stained sample, typically makes a qualitative determination of whether the tumor sample is malignant. It is difficult, however, to ascertain a tumor's aggressiveness merely through histological examination of the sample.

Automated (computer-aided) image analysis systems known in the art can augment visual examination of samples. In a representative system, the magnified image of a cell or tissue sample is exposed to reagents that detect a specific biological marker, and the images processed by a computer that receives the image from a charge-coupled device (CCD) or camera such as a television camera. Such a system can be used, for example, to detect and measure expression levels of the estrogen receptor (ER), the progesterone receptor (PR), or the oncogene HER-2/*neu* in a particular sample. Using this methodology, a more effective regimen of therapy can be administered; for example, hormone therapy can be used where samples test positive for ER and PR, and anti-oncogene receptor therapy can be used where samples test positive for HER-2/*neu* (National Institute of Health Consensus Development Conference: Steroid Receptors in Breast Cancer, 1919, Bethesda, MD; Hancock et al., 1991, Cancer Res. 51:4575-80; Arteaga et al., 1994, Cancer Res. 54:3758-65; Bacus et al., 1997, Anal. Quant. Cytol. Histol. 19:316-28; Sliwkowski et al., 1999, Semin. Oncol. 26:60-70; Shale, 1999, Semin. Oncol. 26:71-77; Cobleigh et al., 1999, J. Clin. Oncol. 17:2639-48). Further, Bacus et al. (Proceedings of the American Association for Cancer Research Annual, vol. 42, page 243, March 2001), describe AKT2 upregulation in Her-2/neu overexpressing breast cancers.

Apoptosis, or programmed cell death that occurs as part of normal mammalian development, was first observed nearly a century ago. The induction of developmental cell death is a highly regulated process that can be suppressed by a variety of extracellular stimuli. For example, the profound biological consequences of growth factor suppression of apoptosis are exemplified by the critical role of target-derived neurotrophins in the survival of neurons and the maintenance of functional neuronal circuits (Pettmann and Henderson, 1998, Neuron 20:633-47). The ability of trophic factors to promote survival is attributed, at least in part, to the phosphatidylinositide 3'-OH kinase (PI3K)/*c-akt* kinase cascade. Several targets of the PI3K/*c-akt* signaling pathway have been identified that may underlie the ability of the regulatory cascade to promote survival and forestall apoptosis. This behavior is relevant to cancer diagnosis because cell survival and apoptosis avoidance is a necessary part of the uncontrolled proliferation characteristic of cancer cells.

Thus, there exists a need in the art to detect cells, particularly cancer cells, that are capable of forestalling apoptosis, as a part of better methods for diagnosing, staging and evaluating cell and tissue specimens obtained from patients, either as part of an initial diagnosis of cancer, in monitoring the efficacy of clinical efforts to control or ablate cancer cells, or for detecting recurrence of a primary tumor or the existence of metastatic disease in a cancer patient. There is specifically a need in the art for improved detection of expression of the products of tumor marker genes for improving diagnoses and making accurate diagnoses as early as possible in the course of the disease.

The invention provides methods for quantifying AKT1 or AKT2 protein expression and activation levels in cells or tissue samples obtained from an animal, most preferably a human cancer patient or an individual suspected of having cancer. Specifically, the invention provides methods for quantifying AKT1 and AKT2 protein expression or activation levels using an imaging system quantitatively. More specifically, the invention provides methods in which an imaging system is used to receive, enhance, and process images of cells or tissue samples, that have been stained with AKT protein-specific stains, in order to determine the amount or activation level of AKT protein expressed in the cells or tissue samples from such an animal. In preferred embodiments of the methods of the invention, a calibration curve of AKT1 and AKT2 protein expression is generated for at least two cell lines expressing differing amounts of AKT protein. The calibration curve is then used to quantitatively determine the amount of AKT protein that is expressed in a cell or tissue sample. Analogous calibration curves can be made for activated AKT1 or AKT2 proteins using reagents specific for the activation features. It can also be used to determine changes in amounts and activation state of AKT (AKT1 and AKT2) before and after clinical cancer treatment.

In one embodiment of the methods of the invention, AKT2 protein expression in a cell or tissue sample is quantified using an enzyme-linked immunoabsorbent assay (ELISA) to determine the amount of AKT2 protein in a portion of a cell pellet prepared from at least two control cell lines expressing differing amounts of AKT2 protein, to which the expression of AKT2 in the cell or tissue sample is compared. In other embodiments of the methods of the invention, the amount of AKT2 protein expressed in a cell or tissue sample is indirectly quantified by hybridization of *c-akt2* "mRNA isolated from the cell or tissue sample to high density oligonucleotide arrays, or determination of *c-akt2* mRNA expression by RT-PCR or by Northern blot hybridization experiments. Importantly, a separate portion of the same cell pellet is then stained with an anti-AKT2 antibody followed by immunohistochemical analysis to determine the amount of AKT2 protein in the sample, preferably by measuring the optical density of the sample at a wavelength specific for the immunohistochemical stain used to detect AKT-2 protein. In the practice of a preferred embodiment of the invention, a calibration curve is prepared in which the concentration of AKT2 protein, most preferably determined by ELISA, is plotted against the optical density measurement for AKT2 protein. In order to determine the amount of AKT2 protein expressed in a cell or tissue sample, the optical density for AKT2 protein is measured and compared with the calibration curve generated from the control cell lines.

Specific preferred embodiments of the present invention will become evident from the following more detailed description of certain preferred embodiments and the claims.

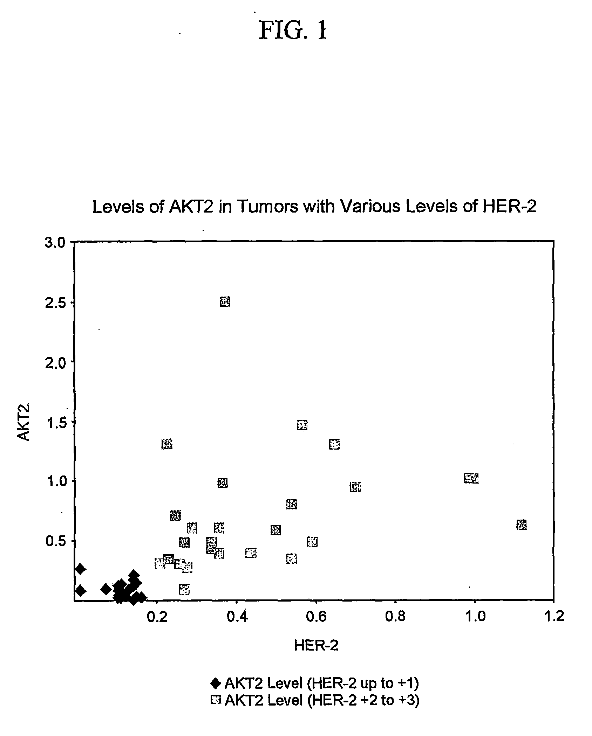

Figure 1 is a chart showing the results of HER-2/*neu* and AKT-2 quantitation by image analysis of breast cancer cells in tumor sections. AKT-2 results are shown in arbitrary units of optical density whereas HER-2/*neu* results are expressed in picograms of HER-2/*neu* protein per cell. Cells that do not overexpress HER-2/*neu* (+1 and negative) are indicated by solid black squares and cells expressing HER-2/*neu* at levels corresponding to +2 and +3 are indicated by gray squares.

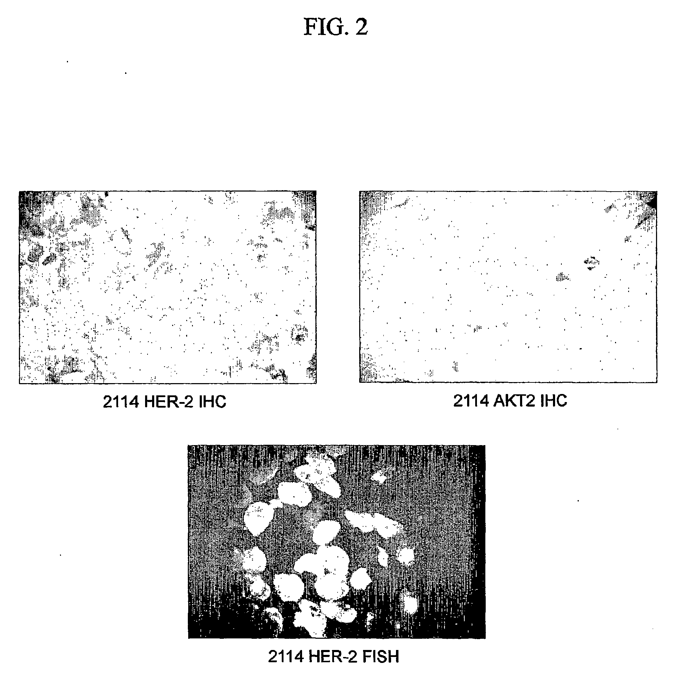

Figure 2 is an immunohistochemical analysis of paraffin sections of breast cancer tissues stained for HER-2/*neu* (2114 HER-2 IHC), for AKT-2 (2114 AKT2 IHC), or by the alkaline phosphatase technique and counterstained by the Feulgen method to provide fluorescent *in situ* hybridization (FISH) analysis (2114 HER-2 FISH).

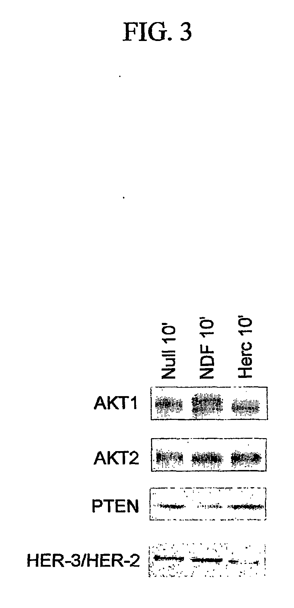

Figure 3 is a Western blot showing levels of AKT1 and AKT2 in phosphorylated and non-phosphorylated forms. Lane 1: negative control; Lane 2: treatment with AKT activator NDF/Heregulin; Lane 3: treatment with herceptin.

This invention provides methods for quantitatively determining expression and activation levels for cellular proteins, AKT1 and AKT2, that are involved in mediating avoidance of apoptosis in tumor cells, particularly human tumor cells as detected in cell or tissue samples from an individual. These methods depend on the identification of AKT proteins as important mediators of apoptosis in normal and tumor cells in mammals, particularly humans

The identification of AKT (the protein product of the *c-akt* gene) as a key regulator of cellular survival has significant implications for oncogenesis and drug resistance. For example, the loss of a human tumor suppressor, PTEN, correlates with increased AKT activity (Li et al., 1997, Science 275:1943-47; Liaw et al., 1997, Nat. Genet. 16:64-67; Nelen et al., 1997, Hum. Mol. Genet. 6:1383-87; Cantley and Neel, 1999, Proc. Natl. Acad. Sci. U.S.A. 96:4240-45; Datta et al., 1999, Genes Dev. 13:2905-27). In addition, suppression of apoptosis is not the only function that AKT may have in promoting oncogenesis. In some circumstances, AKT can also induce cell cycle progression. However, the observation that AKT can suppress apoptosis, considered in light of the finding that cells can be rendered resistant to apoptosis through either the deletion of PTEN, the overexpression of active Ras, or the overexpression of active PI3K, suggests that oncogenes may block adaptive cellular apoptosis by hyperactivating AKT.

Given the complexity of the apoptotic machinery, there are a number of pathways by which AKT might act to promote cell survival and inhibit cell death. AKT may block apoptosis by regulating expression or activity of members of *Bcl-*2 family genes (that are known to play a role in cell survival or cell death). Alternatively, AKT may regulate expression or activity of the caspase family of proteins, or the function of death receptor pathways. The regulatory effect of AKT may be through a direct mechanism - the phosphorylation of components of the apoptotic machinery, *for example* - or an indirect mechanism - such as by altering the expression level of genes that encode components of the death machinery. Recent studies suggest that AKT regulates apoptosis at multiple sites. A number of AKT targets, all playing critical roles in the mediation of cell death, have been identified, including BAD, caspase-9, the Forkhead family of transcription factors, and the NFκB regulator IKK (Datta *et al.,* 1999, *supra*).

The first component of the apoptotic machinery found to be phosphorylated by AKT was the *Bcl-2* family member BAD. BAD was identified on the basis of its ability to bind to BCL-2; analysis of the primary structure of *Bad* revealed that it is similar to *Bcl-2* (Yang et al., 1995, Cell 80:285-91). The finding that AKT suppresses BAD-induced death by direct phosphorylation of BAD is consistent with correlative evidence suggesting that the endogenous PI3K/AKT pathway culminates in the phosphorylation of endogenous BAD. In addition, stimuli such as ceramide, ultraviolet (UV) irradiation, infrared radiation (IR), and sorbitol, that downregulate AKT activity through as-yet-uncharacterized mechanisms, each also inhibit BAD phosphorylation (Zundel and Giaccia, 1998, Genes Dev. 12:1941-46).

AKT also phosphorylates caspase-9. This phosphorylation event has functional consequences, as extracts from cell lines overexpressing AKT block cytochrome C-mediated caspase-9 activation *in vitro.* These results suggest that AKT promotes cell survival through the inactivation of caspase-9 downstream of cytochrome C release. It is not clear how phosphorylation of caspase-9 results in its inactivation, although phosphorylation of caspase-9 appears to inactivate the intrinsic catalytic activity of the protein.

In addition, there is evidence that AKT is involved in mediating the tumorigenic effects of ErbB family gene expression. The ErbB family (HER) of oncogene receptors contains four members: HER (EGFR), HER-2, (ErbB-2), HER-3, and HER-4. Interest in this family of receptors has been greatly stimulated by the finding that overexpression of HER-2 transforms cells, and correlates with an increase in the progression and metastasis of human breast and ovarian cancers. Most recently, a humanized monoclonal antibody to HER-2 has been approved for treatment of breast cancer patients with overexpressed HER-2. Thus the significance of HER-2 overexpression in breast cancer development has been established (Liu et al., 1999, Biochem. Biophys. Res. Commun. 261:897-903).

Heregulin, which is a ligand for both the HER-3 (ErbB-3) and HER-4 (ErbB-4) receptors, can activate HER-2/*neu* through the formation of a heterodimer. Heregulin is also a potent and rapid activator of AKT enzymatic activity in MCF-7 cells that express HER-2. This activation is mediated by HER-2 or HER-3 stimulation of the PI3K pathway. Moreover, overexpression of HER-2 in BT474 breast cancer cells correlates with an increase in the basal activity of AKT. Finally, a monoclonal antibody to HER-2, which is used to treat breast cancer patients, lowers the basal AKT activity in these cells. This implicates the PI3K/AKT pathway as one of the downstream targets of heregulin/HER-2 signaling. Thus, the PI3K/AKT cascade may play a role in the ability of HER-2 overexpression to generate a more aggressive breast cancer phenotype.

AKT2 also plays an important role in cell survival and in blocking programmed cell death following radiation or chemotherapy treatment. For example, AKT2 was found to be overexpressed in pancreatic, breast, and ovarian cancers. In patients where HER-2/*neu* overexpression has been detected, not only is the *c-akt2* gene found to be transcriptionally activated, but the AKT2 protein has also been shown to be overexpressed. In a comparison of MCF-7 cells, which express a basal level of HER-2/*neu*, and MCF-7 cells transfected with a HER-2/*neu* expression construct, *c-alct2* mRNA levels increased 27-fold in the HER-2/*neu* transfected MCF-7 cells. In tumor samples in which HER-2/*neu* was found to be overexpressed, AKT2 was found to be upregulated (Table I).

**Table I**

| **Image analysis quantification of BER-²/*neu* and AKT2 in Breast Cancer Cells** | | |
|---|---|---|
| Sample Number | HER-2/*neu* Results | Akt2 Results |
| 00-98 | 0.01 (neg) | 0.09 |
| 00-020 | 0.01 (neg) | 0.08 |
| 00-024 | 0.01 (neg) | 0.27 |
| 00-074 | 0.07 (neg) | 0.10 |
| 00-248 | 0.10 (+1) | 0.12 |
| 00-365 | 0.10 (+1) | 0.02 |
| 00-373 | 0.10 (+1) | 0.05 |
| 00-388 | 0.10 (+1) | 0.05 |
| 00-52 | 0.10 (+1) | 0.09 |
| 00-302 | 0.11 (+1) | 0.07 |
| 00-201 | 0.11 (+1) | 0.14 |
| 00-376 | 0.11 (+1) | 0.02 |
| 00-392 | 0.11 (+1) | 0.08 |
| 00-221 | 0.11 (+1) | 0.09 |
| 00-51 | 0.12 (+1) | 0.03 |
| 00-319 | 0.12 (+1) | 0.04 |
| 00-117 | 0.13 (+1) | 0.10 |
| 00-49 | 0.14 (+1) | 0.21 |
| 00-407 | 0.14 (+1) | 0.01 |
| 00-380 | 0.14 (+1) | 0.18 |
| 00-79 | 0.14 (+1) | 0.12 |
| 00-299 | 0.15 (+1) | 0.04 |
| 00-332 | 0.15 (+1) | 0.15 |
| 00-250 | 0.16 (+1) | 0.02 |
| 00-28 | 0.16 (+1) | 0.04 |
| 00-07 | 0.16 (+1) | 0.04 |
| 00-202 | 0.21 (+3) | 0.30 |
| 00-386 | 0.23 (+3) | 0.34 |
| 00-162 | 0.23 (+3) | 1.30 |
| 00-163 | 0.25 (+3) | 0.70 |
| 00-352 | 0.26 (+3) | 0.29 |
| 00-377 | 0.27 (+3) | 0.48 |
| 00-04 | 0.27 (+3) | 0.08 |
| 00-316 | 0.28 (+3) | 0.26 |
| 00-123 | 0.29 (+3) | 0.60 |
| 00-411 | 0.34 (+4) | 0.48 |
| 00-412 | 0.34 (+4) | 0.42 |
| 00-288 | 0.36 (+4) | 0.38 |
| 00-278 | 0.36 (+4) | 0.60 |
| 00-153 | 0.37 (+4) | 0.97 |
| 00-154 | 0.38 (+4) | 2.50 |
| 00-308 | 0.44 (+4) | 0.38 |
| 00-349 | 0.50 (+4) | 0.58 |
| 00-08 | 0.54 (+4) | 0.79 |
| 00-314 | 0.54 (+4) | 0.33 |
| 99-2415 | 0.57 (+4) | 1.45 |
| 00-124 | 0.59 (+4) | 0.48 |
| 99-2430 | 0.65 (+4) | 1.29 |
| 00-408 | 0.70 (+4) | 0.93 |
| 00-25 | 0.99 (+4) | 1.00 |
| 00-335 | 1.00 (+4) | 1.00 |
| 00-05 | 1.12 (+4) | 0.61 |

| | | |
|---|---|---|
| AKT2 results are shown in arbitrary units of optical density, whereas HER-2/*neu* results are expressed in picograms of HER-2/*neu* protein per cell. | | |

These results suggest that the activation and overexpression of AKT2 in breast cancers that overexpress HER-2/*neu* may be a significant factor in the aggressive biological behavior of these cancers. Therapeutic agents directed at components of the AKT signaling pathways and diagnostic tests for detecting and measuring the level of AKT proteins and detecting and measuring their activation states are important in the treatment of such cancers. There is a need in the art for a reliable assay for determining AKT protein levels and their activation state in cells or tissue samples obtained from patients. A satisfactory method must also allow the pathologist to exclude normal tissue from the analysis.

In the practice of a preferred embodiment of the methods of this invention, a two-component immunohistochemical staining system is used so that the cell pellets or tissue are counterstained with one color while the proteins of interest in the cell pellet or tissue sample are stained with a different color. The image of the cells in the cell pellet and tissue sample is then magnified in a light microscope and split into a pair of separated images. A pair of optical filters that are specifically matched to have a maximum absorption for each specific stain is used to enhance the separated images. One of the optical filters preferentially transmits light having a wavelength at the absorption wavelength of the counterstained tissue. The other narrow bandpass optical filter preferentially transmits in the regions of spectral absorption for the stain used to detect the protein of interest. Using image analysis filters, different cellular proteins in various components, such as the membrane, cytoplasm and nucleus, can be quantified. For optimal results, the imaging system is calibrated prior to taking any measurements.

The preferred embodiment of the present invention and its advantages are best understood by referring to Examples 1-5. These Examples are illustrative of specific embodiments of the invention, and various uses thereof. They are set forth for explanatory purposes only, and are not to be taken as limiting the invention.

### Example 1

### Image analysis of AKT2 protein expression

Using a two component immunohistochemical staining system as described above, a calibration curve is generated using at least two cell lines expressing differing amounts of AKT2 protein. Cell or tissue samples from human cancer patients are stained in the same manner as the second portion of the control cell pellet and optical densities measuring the extent of immunohistochemical staining are determined for each tissue sample tested. In the preferred embodiment of the method of the present invention, the tissue sample and the second portion of the cell pellet are stained simultaneously to account for any potential differences between the lots of the stains used or variations in the staining process. The amount of biological protein in the tissue sample is determined using the calibration curve generated from measurements of AKT2 protein in the control cell lines.

The average sum optical density per pixel, as calculated in image analysis, takes into account the total optical density of the AKT2 protein positive stained image and divides it by the total number of pixels of the image stained using counterstains such as Fast Green or ethyl green to derive the total pixels comprising the membrane or nuclear area. A calibration curve is generated after each staining.

A calibration curve is derived in order to calibrate the system so that cell pellets can be used as standards to quantify AKT2 protein levels in tissues. The intensity of the color formed by the enzyme reaction in the ELISA as known in the art is proportional to the concentration of the AKT2 protein in the sample, within the working range of the assay. A curve can be obtained by plotting the AKT2 protein concentration (*f*mol/mg from ELISA) or receptors per cell versus the average O.D. data from the image analysis of the stained slides cut from a separate portion of these same three pellets.

### Example 2

### Image analysis of HER-2/neu protein expression

Using HER-2/*neu* as an example, four frozen cell calibrator pellets corresponding to HER-2/*neu* expressing cell, for which the ELISA-determined amount (*f*mol) of HER-2/*neu* per mg protein was known, were cut and stained along with patient's tissue sections in each round of staining. For each tissue, two portions were examined - one embedded in O.C.T. (Optimal Cutting Temperature, Baxter Scientific Products, McGraw Park, IL) for frozen sectioning and another that was ground in liquid nitrogen and placed in homogenization buffer to be used in an ELISA assay. The ELISA portion of each tissue was processed for HER-2/*neu* using the CalBiochem ELISA Kit. These stained slides were then quantitated using image analysis to determine the average O.D. (HER-2/*neu*). The amount of HER-2/*neu* protein expressed in the calibrator cell lines as determined by ELISA and image analysis are shown in Table II.

**Table II**

| **HER-2/*neu* ELISA and HER-2/*neu* Image Analysis (IA) Quantitation Data on Four Cell Pellets** | | |
|---|---|---|
| Pellet | ELISA *f*m/µg | IA *f*m/µg |
| MCF-7 | 0.28 | 0.24 |
| MCF-7 transfected with HER-2 | 0.53 | 0.55 |
| MDA-MB543 | 1.31 | 1.37 |
| SKBR3 | >3.07 | 3.04 |

The average O.D. for each of the calibrator pellets was plotted against the value of HER-2/*neu* per mg protein as derived from the ELISA of the same calibrator pellets. The equation from this graph was then used to derive the amount (*f*mol) of HER-2/*neu* in five patient tissue samples using the average O.D. from the quantitation. Each round of staining of tissue samples was done in conjunction with sections from a set of calibrator pellets. Each time a series of tissues was tested, sections of the pellets were included in the staining run and their quantitated values (average O.D.) used to generate a calibration curve. The amount of HER-2/*neu* protein expressed in the patient tissue samples as determined by ELISA and image analysis are shown in Table III.

**Table III**

| **HER-2/*neu* ELISA and BER-2/*neu* Image Analysis (IA) Quantitation on Five Breast Cancer Tissues** | | |
|---|---|---|
| Tissue Sample | ELISA *f*m/µg | IA *f*m/µg |
| 98-510 | 0.23 | 0.30 |
| 98-511 | 1.74 | 1.12 |
| 98-551 | 0.05 | 0.08 |
| 98-594 | 0.16 | 0.76 |
| 98-664 | 0.17 | 0.20 |

The results of these procedures indicated that this method yields quantitative values for the amounts of HER-2/*neu* expressed by tumor cells. Table I shows preliminary results obtained using antibody units of optical density to determine the levels of AKT2 protein in breast tissue which either express normal (+1) levels HER-2/*neu* or overexpress HER-2/*neu*. A high level of AKT2 protein expression has been detected in HER-2/*neu* overexpressing cancers (+3 or +4). Thus, an automated (computer-aided) image analysis system combined with a series of cell pellets to be used as calibrators presents an accurate method for quantification of AKT2 protein expression levels in cell or tissue samples. The same method can be applied to measuring the amount of AKT activation before and after treatment with a specific drug that blocks AKT activity.

### Example 3

### AKT up-regulation is associated with HER-2/neu over-expression and confers resistance to apoptosis in vitro

MCF7 (obtained from the Michigan Cancer Foundation, Detroit, MI) and MCF7/HER-2/*neu* cells expressing 5-8 fold elevated expression of HER-2/*neu*, which were generated previously by transduction of MCF7 cells with an ErbB-2 cDNA expression vector (Bacus *et al*., 1996; Peles *et al*., 1993; Daly *et al.,* 1997), were grown in RPMI 1640 (Gibco, Grand Island, NY) supplemented with 10% fetal bovine serum, penicillin (100 µg/ml), in a humidified incubator with 8% CO₂ in air at 37°C. The cells were induced to undergo apoptosis under increasing hypoxic conditions in the presence or absence of the PI3, kinase/Akt inhibitor Wortmannin (Calbiochem, San Diego, CA). Cells treated with Wortmannin were exposed to 50 µM of the inhibitor 7-48 hours after cell plating and for 1-3 days thereafter. Under hypoxic conditions, the MCF7 cells were sensitive to apoptosis. The presence of Wortmannin had only a marginal effect on the survival of MCF7 cells under hypoxia. However, the MCF7/HER-2/*neu* cells resisted apoptosis under hypoxia, while the viability of the MCF7/HER-2/*neu* cells exposed to Wortmannin was greatly reduced. Cellular resistance to hypoxia in cells overexpressing HER-2/*neu* is therefore associated with activation of the AKT pathways. The role of PI3 kinase/AKT and HER-2/*neu* pathways in cell survival under hypoxia was confirmed by growing various cell lines in hypoxic conditions and correlating survival with expression of HER-2/*neu*.

MDA-MB-435 and MDA-MB-435/HER-2 cells were obtained from Dr. Yu at the UTMD Anderson Cancer Center, Houston, TX. Cells were grown in RPMI 1640 supplemented as above in 8% CO₂ at 37°C. Western blotting was done as follows. Cells were lysed in lysis buffer (50 mM Tris-HCl, pH 7.4, 150 mM sodium chloride, 1 mM EDTA, 1% Nonidet P-40, 1 mM sodium orthovanadate, 1 mM sodium fluoride, 1 mM phenylmethylsulfonyl fluoride, 2 ug/ml pepstatin, 2 ug/ml leupeptin, 2 ug/ml aprotinin), centrifuged at 4°C for 5 min at 6500g, and protein concentration was determined with a BioRad Protein Assay Kit (BioRad, Hercules, CA). Proteins were separated by electrophoresis using a Bis-Tris NuPAGE system with 1x MOPS running buffer (Novex, San Diego, CA) and transferred to Hybond C-extra membrane (Amersham Pharmacia Biotech, Piscataway, NJ). Membranes were blocked with non-fat dry milk, incubated with the appropriate antibodies, washed with PBS (phosphate buffered saline) and incubated with horseradish peroxidase-conjugated secondary antibody. Detection was carried out with Renaissance Chemiluminescence Reagent Plus (NEN Life Science, Boston, MA). The MDA-MB-435/HER-2 cells over-express HER-2/*neu* 10-fold compared with the parental MDA-MB-435 cell line as determined by Western blot analysis. Lysates from these cells were collected and AKT-2 expression was examined by Western blot analysis with two different antibodies against AKT-2, a polyclonal antibody (Santa Cruz, CA) and a monoclonal antibody (Dr. Testa, Fox Chase Cancer Center, Philadelphia, PA). An increase in the levels of AKT-2 was observed in the cells over-expressing HER-2/*neu*.

### Example 4

### AKT-2 up-regulation correlates with over-expression of HER-2/neu in cancer

Tumor samples from 42 patients were examined for *c-akt-1* and *c-akt-2* expression. The tissues were divided into two groups: those expressing normal levels (up to +1) of HER-2/*neu* and those over-expressing (from +2 to +3) HER-2/*neu*. Tissues were stained for AKT- and AKT-2. While levels of AKT- were similar in both groups, image analysis revealed that AKT-2 was up-regulated up to 10-fold in the HER-2/*neu* over-expressing tissues (Figure 1). Figure 2 shows by immunohistochemical analysis that high expression of AKT-2 correlates with overexpression of HER-2/*neu* in a paraffin section of a breast cancer tumor.

### Example 5

### Activation of c-akt in cells over-expressing HER-2/neu

The activated (phosphorylated) state of total AKT (AKT-1 plus AKT-2) in cells over-expressing HER-2/*neu* was measured to study the functional significance of *c-akt* in HER-2/*neu* over-expressing cells and cancers. SKBR3, BR474, and AU565 cells (Cell Culture Laboratory Navy Biosciences Laboratory, Navy Supply Center, Oaldand, CA), all of which over-express HER-2/*neu*, were treated with the known *c-akt* activator, NDF/Heregulin (10 ng/ml; a gift of Y. Yarden, Rehovot, Israel), or the monoclonal antibody against HER-2/*neu*, Herceptin (20 mg/ml), 7-48 hours after plating and continued for 1-3 days. Cell lysates from the SKBR3, BR474, and AU565 cells were examined by Western blot analysis as described above. Activated AKT was determined by antibodies that specifically recognize the phosphorylated state of AKT protein. Cells were lysed as above and subjected to immunoprecipitation with anti-AKT-1 and anti-AKT-2 antibodies Immunoprecipitation was carried out as follows. Cell lysates (250 ug protein per 500 ul buffer) were pre-incubated with protein A + protein G agarose beads (Gibco BRL Life Technologies, Rockville, MD), incubated with 0.5 ug of anti-AKT-1 or anti-AKT-2 antibodies (Upstate Biotechnology, Lake Placid, NY) overnight at 4°C, followed by incubation for 1 hour with 40 ul of protein A + protein G agarose beads. Immune complexes were washed with cold lysis buffer and examined by Western blotting with anti-phospho-Akt to detect phosphorylated forms of AKT-1 or AKT-2 (Figure 3).

The total amount of AKT as well as AKT-1 phosphorylation was increased in cells treated with Heregulin. Treatment with Herceptin decreased the phosphorylation of total AKT and AKT-1. The basal level of AKT-2 phosphorylation was high and remained high after treatment with either Heregulin or Herceptin. Exposure to Herceptin and Heregulin for 3 days resulted in down-regulation of the phosphorylated state of AKT-1 but did not affect AKT-2. Therefore, activation of the HER-2/Her-3 receptor by NDF/Heregulin leads to activation of AKT and AKT-1. Treatment with Herceptin, which leads to down-regulation of HER-2/*neu*, also leads to down-regulation of AKT-1, but not AKT-2. To determine if hetero-dimerization of HER-2 and HER-3 plays, a role in AKT-1 activation, HER-3 was immunoprecipitated from untreated NDF and Herceptin treated MDA-MB-474 cells. Immunoprecipitates were analyzed by Western blot with anti-HER-2 antibodies. Down-regulation of AKT activation correlated with down-regulation of HER-2/Her-3 heterodimers. Treatment with Herceptin was associated with down-regulation of these heterodimers, which resulted in down-regulation of AKT-1 activity. Basal levels of phosphorylated AKT-2 were unaffected by treatment with Heregulin or Herceptin.

## Claims

1. A method for determining the quantify of AKT protein, the AKT protein activation level or both in a cell or tissue sample, the method comprising the steps of:
(a) immunohistochemically staining AKT protein in a plurality of control cell pellets using a detectably labeled antibody directed against AKT, wherein the quantity of AKT protein, the AKT protein activation level or both in the plurality of control cell pellets is independently known, and wherein the expression, activation level or both expression and activation level of AKT in each of the plurality of control cell pellets is not the same;
(b) determining an average optical density of stained AKT protein per pixel of cellular area for each of the stained plurality of control cell pellets in (a);
(c) generating a calibration curve relating the known quantity of AKT protein, AKT protein activation level or both with said average optical density of stained AKT protein per pixel of cellular area as determined in (b) for each of the plurality of control cell pellets;
(d) immunohistochemically staining AKT protein from said cell or tissue using said detectably labeled antibody directed against AKT protein;
(e) determining an average optical density of stained AKT protein per pixel of cellular area in said cell or tissue sample;
(f) determining the quantity of AKT protein, the AKT protein activation level or both in said cell or tissue sample by comparing the average optical density of stained AKT protein per pixel of cellular area as determined in step (e) in said biological sample to the calibration curve as generated in step (c), wherein the quantity of AKT protein, the AKT protein activation level or both is derived from the calibration curve.

2. The method of claim 1, wherein the expression, activation level or both expression and activation levels of AKT2 in each of the plurality of control cell pellets is not the same.

3. The method of claim 1, wherein the expression, activation level or both expression and activation levels of AKT1 in each of the plurality of control cell pellets is not the same.

4. The method of claim 2 or 3, wherein the quantity of AKT protein, the AKT protein activation level, or both from each of the control cell pellets is determined by enzyme-linked immunosorbent assay (ELISA).

5. The method of claim 2 or 3, wherein the quantity of AKT protein, the AKT protein activation level, or both from each of the control cell pellets is determined by hybridization of high density oligonucleotide arrays with cellular mRNA or cDNA prepared therefrom,

6. The method of claim 2 or 3, wherein the quantity of AKT protein, the AKT protein activation level, or both from each of the control cell pellets is determined by RT-PCR of cellular RNA or mRNA.

7. The method of claim 2 or 3, wherein the quantity of AKT protein, the AKT protein activation level, or both from each of the control cell pellets is determined by Northern blot hybridization.

8. The method of claim 2 or 3, wherein the quantity of AKT protein, the AKT protein activation level, or both from each of the control cell pellets is determined by protein microarray.

## Patentansprüche

1. Verfahren zur Bestimmung der Menge von AKT-Protein, des AKT-Protein-Aktivierungsgrads, oder von beiden in einer Zell- oder Gewebeprobe, wobei das Verfahren die Schritte umfasst:
(a) immunhistochemisches Färben von AKT-Protein in einer Vielzahl von Kontroll-Zellpellets, unter Verwendung eines detektierbar markierten Antikörpers, der gegen AKT gerichtet ist, wobei die Menge an AKT-Protein, der AKT-Protein-Aktivierungsgrad oder beide in der Vielzahl von Kontroll-Zellpellets unabhängig voneinander bekannt ist, und wobei die Expression, der Aktivierungsgrad oder sowohl Expression als auch Aktivierungsgrad von AKT in jedem der Vielzahl von Kontroll-Zellpellets nicht gleich ist,
(b) Bestimmen einer durchschnittlichen optischen Dichte von gefärbtem AKT-Protein pro Pixel des zellulären Bereichs für jede der gefärbten Vielzahl von Kontroll-Zellpellets in (a),
(c) Erzeugen einer Kalibrationskurve, welche die bekannte Menge von AKT-Protein, AKT-Protein-Aktivierungsgrad oder beidem mit der durchschnittlichen optischen Dichte von gefärbtem AKT-Protein pro Pixel des zellulären Bereichs wie in (b) bestimmt für jedes der Vielzahl von Kontroll-Zellpellets in Beziehung setzt,
(d) immunhistochemisches Färben von AKT-Protein aus der Zelle oder dem Gewebe unter Verwendung des detektierbar markierten Antikörpers, der gegen AKT-Protein gerichtet ist,
(e) Bestimmen einer durchschnittlichen optischen Dichte von gefärbtem AKT-Protein pro Pixel des zellulären Bereichs in der Zell- oder Gewebeprobe,
(f) Bestimmen der Menge von AKT-Protein, des AKT-Protein-Aktivierungsgrads oder von beiden in der Zell- oder Gewebeprobe durch Vergleichen der durchschnittlichen optischen Dichte von gefärbtem AKT-Protein pro Pixel des zellulären Bereichs wie in Schritt (e) in der biologischen Probe bestimmt mit der Kalibrationskurve wie in Schritt (c) erzeugt, wobei die Menge von AKT-Protein, der AKT-Protein-Aktivierungsgrad oder beide aus der Kalibrationskurve hergeleitet werden.

2. Verfahren nach Anspruch 1, wobei die Expression, der Aktivierungsgrad oder sowohl Expression als auch Aktivierungsgrad von AKT2 in jedem der Vielzahl von Kontroll-Zellpellets nicht gleich ist.

3. Verfahren nach Anspruch 1, wobei die Expression, der Aktivierungsgrad oder sowohl Expression als auch Aktivierungsgrad von AKT1 in jedem der Vielzahl von Kontroll-Zellpellets nicht gleich ist.

4. Verfahren nach Anspruch 2 oder 3, wobei die Menge von AKT-Protein, der AKT-Protein-Aktivierungsgrad oder beide von jedem der Kontroll-Zellpellets durch Enzym-gekoppelten Immunadsorptionstest (ELISA) bestimmt wird.

5. Verfahren nach Anspruch 2 oder 3, wobei die Menge von AKT-Protein, der AKT-Protein-Aktivierungsgrad oder beide von jedem der Kontroll-Zellpellets durch Hybridisierung von sehr dichten Oligonukleotid-Arrays (high density oligonucleotide arrays) mit zellulärer mRNA oder cDNA, die daraus hergestellt wird, bestimmt wird.

6. Verfahren nach Anspruch 2 oder 3, wobei die Menge von AKT-Proteinen, der AKT-Protein-Aktivierungsgrad oder beide von jedem der Kontroll-Zellpellets durch RT-PCR von zellulärer RNA oder mRNA bestimmt wird.

7. Verfahren nach Anspruch 2 oder 3, wobei die Menge von AKT-Protein, der AKT-Protein-Aktivierungsgrad oder beide von jedem der Kontroll-Zellpellets durch Northern-Blot-Hybridisierung bestimmt wird.

8. Verfahren nach Anspruch 2 oder 3, wobei die Menge von AKT-Protein, der AKT-Protein-Aktivierungsgrad oder beide von jedem der Kontroll-Zellpellets durch einen Protein-Mikroarray bestimmt wird.

## Revendications

1. Procédé pour déterminer la quantité de protéine AKT, le niveau d'activation de protéine AKT ou les deux dans une cellule ou un échantillon de tissu, le procédé comprenait les étapes de:
(a) marquage immunohistochimique de la protéine AKT dans une pluralité de culots cellulaires témoins en utilisant un anticorps marqué de façon détectable dirigé contre l'AKT, dans lequel la quantité de protéine AKT, le niveau d'activation de protéine AKT ou les deux dans la pluralité de culots cellulaires témoins sont indépendamment connus, et dans lequel le niveau d'expression, le niveau d'activation ou les niveaux à la fois d'expression et d'activation de l'AKT dans chacun de la pluralité de culots cellulaires témoins ne sont pas les mêmes ;
(b) détermination d'une densité optique moyenne de protéine AKT marquée par pixel de superficie cellulaire pour chacun de la pluralité marquée de culots cellulaires témoins dans (a) ;
(c) génération d'une courbe d'étalonnage mettant en relation la quantité de protéine AKT, le niveau d'activation de protéine AKT, ou les deux, connus, avec ladite densité optique moyenne de protéine AKT marquée par pixel de superficie cellulaire telle que déterminée en (b) pour chacun de la pluralité de culots cellulaires témoins ;
(d) marquage immunohistochimique de la protéine AKT provenant de ladite cellule ou dudit tissu en utilisant ledit anticorps marqué de façon détectable dirigé contre la protéine AKT ;
(e) détermination d'une densité optique moyenne de protéine AKT marquée par pixel de superficie cellulaire dans ladite cellule ou ledit échantillon de tissu ;
(f) détermination de la quantité de protéine AKT, du niveau d'activation de protéine AKT, ou des deux, dans ladite cellule ou ledit échantillon de tissu en comparant la densité optique moyenne de protéine AKT marquée par pixel de superficie cellulaire telle que déterminée à l'étape (e) dans ledit échantillon biologique avec la courbe d'étalonnage telle que générée à l'étape (c), dans lequel la quantité de protéine AKT, le niveau d'activation de protéine AKT, ou les deux, sont obtenus à partir de la courbe d'étalonnage.

2. Procédé selon la revendication 1, dans lequel le niveau d'expression, le niveau d'activation ou les niveaux à la fois d'expression et d'activation de l'AKT2 dans chacun de la pluralité de culots cellulaires témoins ne sont pas les mêmes.

3. Procédé selon la revendication 1, dans lequel le niveau d'expression, le niveau d'activation ou les niveaux à la fois d'expression et d'activation de l'AKT1 dans chacun de la pluralité de culots cellulaires témoins ne sont pas les mêmes.

4. Procédé selon la revendication 2 ou 3, dans lequel la quantité de protéine AKT, le niveau d'activation de protéine AKT, ou les deux de chacun des culots cellulaires témoins sont déterminés par méthode immunoenzymatique par dosage d'immunosorption liée à une enzyme (ELISA).

5. Procédé selon la revendication 2 ou 3, dans lequel la quantité de protéine AKT, le niveau d'activation de protéine AKT, ou les deux de chacun des culots cellulaires témoins sont déterminés par hybridation d'ensembles d'oligonucléotides haute densité avec de l'ARNm ou de l'ADNc cellulaire préparé à partir de ceux-ci.

6. Procédé selon la revendication 2 ou 3, dans lequel la quantité de protéine AKT, le niveau d'activation de protéine AKT, ou les deux de chacun des culots cellulaires témoins sont déterminés par RT-PCR d'ARN ou d'ARNm cellulaire.

7. Procédé selon la revendication 2 ou 3, dans lequel la quantité de protéine AKT, le niveau d'activation de protéine AKT, ou les deux de chacun des culots cellulaires témoins sont déterminés par hybridation par buvardage de northern.

8. Procédé selon la revendication 2 ou 3, dans lequel la quantité de protéine AKT, le niveau d'activation de protéine AKT, ou les deux de chacun des culots cellulaires témoins sont déterminés par puce à protéines.
